# EUROPEAN PATENT SPECIFICATION

(11) **EP 2 046 287 B1**
(45) Date of publication and mention of the grant of the patent: **23.11.2011**
(21) Application number: 07810662.2
(22) Date of filing: 20.07.2007
(51) Int. Cl.: A61K 9/00, A61P 27/02

(54) **METHODS AND COMPOSITIONS FOR THE TREATMENT AND PREVENTION OF INFECTIONS**
VERFAHREN UND ZUSAMMENSETZUNGEN ZUR BEHANDLUNG UND PRÄVENTION VON INFEKTIONEN
MÉTHODES ET COMPOSITIONS POUR LE TRAITEMENT ET LA PRÉVENTION D'INFECTIONS

(30) Priority: 21.07.2006 US 490917
(43) Date of publication of application: 15.04.2009
(73) Proprietor: Advanced Vision Research, Inc., Woburn, MA 01801 (US)
(72) Inventor: GILBARD, Jeffrey, P., Weston, MA 02493 (US)
(74) Representative: Duffy, Assumpta Dympna
(86) International application number: PCT/US2007/016485
(87) International publication number: WO 2008/011164

(56) References cited:
- WO-A2-96/02264
- FR-A1- 2 558 062
- JP-A- 2003 128 584
- JP-A- 2003 137 781
- US-A- 4 499 077
- US-A1- 2005 028 953
- US-A1- 2005 196 370
- DATABASE WPI Week 200364, Derwent Publications Ltd., London, GB; AN 2003-674170, XP008102790 & JP 2003 128549 A (TSUJI) 08 May 2003

## Description

### BACKGROUND OF THE INVENTION

A condition known as dry eye causes chronic eye irritation resulting from decreased tear production or increased evaporation that results in a loss of water from the tear film and an increase in tear film osmolarity. This increase in tear film osmolarity results in an osmotic dehydration of the surface associated with a decrease in the density of conjunctival goblet cells. Recently it has been shown that dry eye patients have increased bacterial colonization of their eyelids, and that the bacteria found in these patients decrease the proliferation of conjunctival goblet cells in tissue culture ((Graham et al Analysis of Bacterial Flora in Dry Eye, Ocular Surface, 3(1):S68, 2005). Dry-eye patients are also known to be more prone to eye infections, especially in the context of contact lens wear (Lemp MA "Is the dry eye contact lens wearer at risk?", Cornea (United States), 1990, 9 Suppl 1 pS48-50; discussion S54).

Punctal plugs are a frequently used treatment for dry eye. They provide symptomatic relief for patients with dry eye, reduce elevated tear film osmolarity in the disease and reduce ocular surface staining. A problem with punctal plugs is that they are frequently colonized by pathogenic noncomensals, including Pseudomonas aeruginosa and Staphylococcus aureus, that may cause symptoms and increase the risk of eye infections (Soukiasian SH Microbiology of Explanted Punctal Plugs, ARVO Annual Meeting, Program#/Poster# 4981/B305, April 29, 2004; Sugita J, Yokoi N, Fullwood NJ, et al. "The detection of bacteria and bacterial biofilms in punctal plug holes", Cornea (United States), May 2001, 20(4) p362-5).

Another condition of clinical significance is inflammation of the eyelids and the eye surface. This inflammatory disorder, frequently resulting in symptoms of eye irritation, is called blepharitis. In a study involving 332 patients with blepharitis and 160 normal controls, it has been shown that blepharitis patients have greater quantities of bacteria on their eyelids compared to normal controls. This finding applied to patients with both anterior and posterior blepharitis (Groden LR, Murphy B, Rodnite J, et al. "Lid flora in blepharitis", Cornea (United States), Jan 1991, 10(1) p50-3). Bacterial overgrowth has been hypothesized to contribute to the symptoms of blepharitis by the production of bacterial lipases and esterases that hydrolyze the wax and sterol esters in meiburn, creating free fatty acids that are irritating to ocular tissue and may effect tear film stability (Ta CN, Shine WE, McCulley JP, et al. 'Effects of minocycline on the ocular flora of patients with acne rosacea or seborrheic blepharitis', Cornea (United States), Aug 2003, 22(6) p545-8). In addition, these fatty acids may promote eyelid and ocular surface inflammation (Shine WE, McCulley JP, Pandya AG 'Minocycline effect on meibomian gland lipids in meibomianitis patients', Exp Eye Res (England), Apr 2003, 76(4) p417-20).

Eye hygiene, including eye lid cleaning, has been recommended for all of these conditions or circumstances by eye doctors. Many of the treatments available for dry eye conditions are formulated as drops or ointments. However, drops and ointments have a limited lifespan once opened as they commonly become infected with microbial growth.

Chlorine dioxide has been called the ideal biocide. Despite the many advantages of chlorine dioxide, commercial use is limited because it is an unstable, highly reactive gas which is soluble in and decomposes in water. See, e.g., U.S. Pat. No. 4,941,917. Therefore, it has heretofore been necessary to generate aqueous chlorine dioxide solutions on site for immediate use or use within a relatively short time (typically less than an hour). Due to these limitations, chlorine dioxide has not met its full potential as an antimicrobial agent. Similarly, other oxidizing antimicrobial agents such as sodium perborate have been under utilized because of stability and effectiveness issues.

Therefore, a need exists for a preparation that can be used, for example, in or around the eye and is stable for extended periods of time.

### SUMMARY OF THE INVENTION

The instant invention is based on the discovery that eye care preparations comprising chlorine dioxide and a heterocyclic compound, wherein the heterocyclic compound is a xanthine, i.e., dyphylline, exhibit beneficial effects. For example, preparations comprising chlorine dioxide and dyphylline are useful for the treatment and prevention of microbial infection and preservation of ocular products. The addition of chlorine dioxide and a xanthine preserve the solution for extended periods of time. Moreover, these preparations resist microbial growth prior to and after being used by an individual.

Accordingly, in at least one aspect, the instant invention provides eye care preparations comprising chlorine dioxide and a xanthine that improves the antibacterial effect of the chlorine dioxide preparation. The xanthine can be caffeine, theophylline, dyphylline, theobromine, xanthine, xanthinol, methylxanthine, and aminophylline. In a preferred embodiment, the xanthine is dyphylline.

The chlorine dioxide in the preparations can be present in a concentration of about 25-125 ppm, about 50-75 ppm, or about 60 ppm.

The xanthine such as dyphylline can be present in 0.10-10%, 0.25-5% or about 1% to about 3%.

The preparations of the invention include solutions, creams, pastes, ointments, and gels. The preparations can be, for example, topical preparations that are applied to the eye, eye-lid, or eye margin. Further, the preparations are effective against bacteria, fungi, and/or yeast. The preparations of the invention may further comprise a pharmaceutically acceptable carrier.

In one exemplary embodiment, the preparation is a solution. The solutions of the invention can have an osmolality of 180 mOsm/Kg or less, or 165 mOsm/Kg or less.

An exemplary preparation of the invention is an eye care preparation having an osmolality of 165 mOsm/Kg or less and a chlorine dioxide concentration of about 50-75 ppm.

Another exemplary preparation of the invention comprises a solution comprising chlorine dioxide and dyphylline, wherein the chlorine dioxide is present at about 55-75 ppm and the preparation has a total osmolality of 165 mOsm/Kg or less.

Yet another exemplary preparation of the invention comprises, an eye drop comprising chlorine dioxide and dyphylline, wherein the chlorine dioxide is present at about 55-75 ppm and the preparation has a total osmolality of 165 mOsm/Kg or less.

The instant invention also provides methods of using the described preparations. For example, the invention provides a method of preventing or treating microbial infection in a subject by applying the preparations described herein to the infected area in an amount sufficient to prevent or treat a microbial infection in a subject. Exemplary microbial infections include bacterial, fungal and yeast infections.

The invention also provides methods of preventing or treating an infection of the ocular surface in a subject by applying the preparation to an ocular surface in an amount sufficient to prevent or treat an infection of the ocular surface in the subject. An exemplary infection of the ocular surface includes bacterial conjunctivitis. The invention also provides a method for reducing the risk of infection in patients, including dry-eye patients, wearing contact lenses.

The invention also provides methods of reducing the risk of infection of the eye in an eye surgery patient by applying the preparations described herein to an eyelid or ocular surface prior to a surgical procedure in an amount sufficient to reduce the risk of infection in the eye of a surgical patient. In accordance with this method, the preparation is applied multiple times over a number of days preceding the surgery.

The invention also provides methods of reducing the risk of infection of the eye in a subject wearing a punctal plug by applying the preparation described herein to the eye surface, the eyelid margin, the eyelid or the punctal plugs or into the tear film in an amount sufficient to reduce the risk of infection in the eye of the subject wearing a punctal plug.

The invention also provides methods for treating or reducing the risk of infection in a subject by applying the topical preparation described herein to the area that is infected or at risk of becoming infected in an amount sufficient to treat or reduce the risk of infection.

The invention also provides methods of reducing colonization or of treating an infection of the mucosal tissue or the epithelial tissue in a subject by applying the preparation described herein to the mucosal tissue or the epithelial tissue in an amount sufficient to treat the infection in the subject.

The invention also provides a method of treating dry eye in a subject by applying the eye care preparation described herein to the eye or eyelid in an amount sufficient to treat dry eye in the subject.

The invention also provides a method of increasing goblet cell density and mucosal epithelial membrane mucin expression in a subject by applying the eye care preparation described herein to the eye in an amount sufficient to increase goblet cell density and mucosal epithelial membrane mucin expression. This increase can be determined using rose Bengal or other vital staining techniques. In dry eye, the ocular surface stains less with an increase in conjunctival goblet cell density and mucosal epithelial membrane mucin expression.

The invention also provides methods for sterilizing a surface by contacting the surface with the preparation described herein, or incorporating within a surface the preparation described herein, in an amount sufficient to sterilize the surface. Exemplary surfaces include surfaces on a body, a medical instrument, or a medical device. An exemplary medical device includes a contact lens.

The invention also provides kits for the treatment or prevention of a microbial infection, comprising the preparation described herein and instruction for use. The invention also provides kits for the treatment of an ocular disorder comprising the antimicrobial preparation described herein and instruction for use. The kits may further comprise an applicator.

### DETAILED DESCRIPTION OF THE INVENTION

At present, there exists a need for compositions and methods for preventing microbial growth and treating microbial infections. In particular, there exists a need for compositions for preventing microbial growth in products for use in the eye or surrounding area. In particular, the need exists for compositions that remain free of microbial growth for extended periods of time, i.e., compositions that contain a preservative that inhibit microbial growth and that do not irritate the area to which it is applied. Preferably, the compositions will contain an antimicrobial composition that will kill or retard the growth of microbes (e.g., a bactericidal or bacteriostatic composition). In certain embodiments, the compositions are also useful for treating or preventing infection of, for example, the skin. In further embodiments, the preparations of the invention are useful in treating or preventing infection on surfaces, medical devices, and medical instruments.

### Definitions

The invention will be described with reference to the following definitions that, for convenience, are collected here.

The term "cleaning an eyelid" is used herein to describe the act of significantly reducing the amount of dirt, debris, or bacteria, from an eyelid.

The term "dry eye" is known in the art as a condition of a subject that has a loss of water from the tear film due to either a decrease in tear production or an increase in tear film evaporation. Tear production can decrease from lacrimal gland disease, including, but not limited to, that which occurs in Sjögren's syndrome, or from anything that decreases corneal sensation. Examples of conditions that decrease corneal sensation include, but are not limited to, diabetes, long-term contact lens wear, and corneal surgery, including LASIK eye surgery. Tear film evaporation can increase from meibomian gland dysfunction, that manifests itself by stenosis or closure of the meibomian gland orifices, or in the presence of large palpebral fissure widths. Causes for large palpebral fissure width include, but are not limited to, normal biological variation and thyroid eye disease. Dry eye is often an age related disease, and may also be caused by a dietary deficiency of omega-3 essential fatty acids. Dry eye is associated with bacterial overcolonization of the eyelids.

The term "eyelid" as used herein, includes the tarsal conjunctival surface, both the interior and exterior surfaces of the eyelid, the eyelid margin, the glands in and around the eyelid margins, the hair follicles of the eyelid, the eyelashes, and the periocular skin surrounding the eye.

The term "eye surface inflammatory disorder," as used herein, is intended to include disorders associated with eye surface inflammation. These disorders include dry eye, where ocular surface inflammation has been demonstrated, as well as both anterior and posterior blepharitis. In anterior blepharitis, the inflammation is centered around the eyelashes. Posterior blepharitis or meibomitis is associated with inflammation of the tarsal and bulbar conjunctiva, and complicated by hordeolums and chalazions, and leads to meibomian gland dysfunction. Both anterior and posterior blepharitis are associated with bacterial overcolonization of the eyelids.

Animal models with combined dry eye and eye surface inflammatory disorder have been produced, and can be used to test the efficacy of the antimicrobial preparations provided herein. For example, a rabbit model for meibomianitis and meibomian gland dysfunction has been developed. In this animal model, meibomian gland orifice closure results in the development of inflammation around the meibomian glands (i.e., meibomianitis), inflammation in the eyelids (blepharitis), inflammation in the conjunctiva (conjunctivitis) and in an increase in tear film osmolarity and a decrease in the levels of corneal glycogen and conjunctival mucus-containing goblet cells characteristic of dry-eye surface disease.

The term "eyelid disorder" is defined as a disorder that results in inflammation of the eyelashes and/or eyelash follicles and/or eyelid margins, or inflammation of the lipid producing glands that are located in the eyelid, including meibomianitis and anterior blepharitis. Exemplary eyelid disorders include, but are not limited those caused by bacterial infection.

The term "ocular disorder" as used herein, includes ocular surface disorders, disorders of the eyeball, periocular skin disorders, and eyelid disorders. Exemplary ocular disorders include, but are not limited to, dysfunctions of the tear film, inflammation of the eyelid margins due to bacterial infection, infections inside the eye known as endophthalmitis, and dry eye.

The term "treatment" as used herein is defined as prophylactic treatment (e.g., daily preventative use) or therapeutic treatment (e.g., a single treatment or a course of treatment) of a subject with or at risk for an ocular disorder, or with an ear or skin condition, that are associated with or exacerbated by infections or bacterial colonization.

The term "preparation or antimicrobial preparation" as used herein includes compositions comprising chlorine dioxide, and a xanthine that stabilizes the preparation. The preparation of the invention can be a solution, cream, paste, ointment, gel or the like. The preparations of the invention can be applied to, for example, the skin, eye, or eyelid.

The term "heterocyclic compound that improves the antibacterial effect of the chlorine dioxide preparation" is intended to include a xanthine that, when combined with chlorine dioxide, provide beneficial effects. For example, the heterocyclic compound may increase the half-life of the chlorine dioxide in the preparation, may provide beneficial treatment effects, may increase the efficacy of the preparation, etc. In an exemplary embodiment, the heterocyclic compound improves the bactericidal effect of chlorine dioxide in the compositions, thereby extending the time that the composition is free of microbial growth. Specific exemplary heterocyclic compounds include caffeine, theophylline, dyphylline, theobromine, xanthine, xanthinol, methylxanthine, and aminophylline. In a preferred embodiment, the heterocyclic compound used in the preparations of the invention is dyphylline.

As used herein the language "pharmaceutically acceptable carrier" is intended to include any and all solvents, agents, and the like, compatible with pharmaceutical administration. The use of such media and agents for pharmaceutically active substances is well known in the art. Except insofar as any conventional media or agent is incompatible with the antimicrobial preparations described herein, such media can be used in the compositions of the invention. Pharmaceutical compositions suitable for topical application preferably take the form of a drop, solution, ointment, cream, lotion, paste, gel, spray, aerosol, or oil. Exemplary carriers which may be used include water, carboxymethylcellulose, petroleum jelly, mineral oil, lanolin, polyethylene glycols, alcohols, and combinations of two or more thereof.

### Methods and Compositions

Effective health and cleanliness of an eye is dependant upon the ability to control the level microbes. Accordingly, compositions that have extended utility once opened, i.e., that resist microbial growth, are useful for the treatment of the eye.

The present invention provides compositions and methods, which decrease, e.g., significantly decrease, the number of microbes present in or around, for example, an eye, or in materials used in or around the eye.

Accordingly, the invention is directed to a preparation comprising an antibacterial concentration of chlorine dioxide and a xanthine that improves the antibacterial effect of the chlorine dioxide preparation. The preparation may also contain a pharmaceutically acceptable carrier or water. The preparation may be used as a preservative for materials used in conjunction with the eye such as eye drops or may be specifically formulated for the treatment of a particular disorder, e.g., an ocular disorder selected from blepharitis, dry eye, infectious conjunctivitis, an ear infection, or a skin infection. The preparation may also be used to sterilize, for example, surgical instruments, medical indwelling devices, surfaces and the like. The preparation may also be incorporated into the surface of medical devices for sustained release of the preparation. One of skill in the art would understand that the preparation of the invention may be prepared in the form of drops, solution, paste, cream, foam, gel, ointment, or the like, or incorporated into sustained-release carriers such as sustained-release polymers, liposomes and microcapsules.

There are several commercial generators for producing the chlorite/chlorine chlorine dioxide. Suitable generators are disclosed in U.S. Pat. Nos. 4,247,531; 5,204,081; 6,468,479; and 6,645,457.

Chlorine dioxide can be produced with high efficiency by reducing sodium chlorate in a strong acid solution with a suitable reducing agent (for example, hydrogen peroxide, sulfur dioxide, or hydrochloric acid):

2ClO₃⁻+2Cl⁻+4H⁺ → 2ClO₂ + Cl₂ + 2H₂O

Alternatively, chlorine dioxide can be made by one of three methods using sodium chlorite: The sodium chlorite - chlorine gas method (2 NaClO₂ + Cl₂ → 2 ClO₂ + 2 NaCl); the sodium chlorite - hypochlorite method (2 NAClO₂ + 2 HCl + NaOCl → 2 ClO₂ + 3 NaCl + H₂O); or the sodium chlorite - hydrochloric acid method (5NaClO₂ + 4HCl → 5NaCl + 4ClO₂). Finally, chlorine dioxide can be produced by electrolysis of a chlorite solution (NaClO₂ + H₂O → ClO₂ + NaOH + 1/2 H₂). Preparations of the invention comprise between about 25-200 ppm of chlorine dioxide, about 50-150 ppm of chlorine dioxide, about 50-100 ppm of chlorine dioxide, or about 50-75 ppm of chlorine dioxide. For treatment of infection, higher values may be used.

The heterocyclic compound such as dyphylline can be present in 0.10-10%, 0.25-5% or about 1% to about 3%.

In certain embodiments, the antimicrobial preparation is an aqueous solution containing chlorine dioxide as described herein. The solutions of the invention can have an osmolality of, for example, about 180 mOsm/Kg, about 175 mOsm/Kg or less, about 170 mOsm/Kg, about 165 mOsm/Kg or less, about 160 mOsm/Kg or less, or about 155 mOsm/Kg or less.

An exemplary preparation of the invention is a preparation having an osmolality of 165 mOsm/Kg or less and a chlorine dioxide concentration of about 50-75 ppm.

The preparations may further include buffers, solubilizers, viscosity increasing agents, preservatives, anti-inflammatory agents and salts.

In a preferred embodiment, the invention provides an eye drop comprising chlorine dioxide and a heterocyclic compounds. The eye drop includes a balance of electrolytes found in natural tear fluid required for ocular surface maintenance, function and repair. These electrolytes are present in amounts and proportions sufficient to maintain or restore conjunctival goblet cells and corneal glycogen, thereby maintaining mucus- mediated lubrication and the potential for normal healing. This enables topical application of the preparation to ocular surfaces preferably without substantially reducing the density of conjunctival mucus-containing goblet cells or levels of corneal glycogen. Goblet cells form a critical layer of the tear film, providing the eye surface with lubrication, and playing an important role in the system that traps foreign matter that may enter the eye, and promptly removes it. Corneal glycogen is the energy source for the sliding step in corneal wound healing. Their preservation is therefore important in maintaining the health of ocular surfaces.

The eye drop compositions of the invention include, in addition to chlorine dioxide and a heterocyclic compounds, e.g., dyphylline, a balance of electrolytes naturally found in tear fluid. These electrolytes principally include major amounts of sodium and chloride, and lesser amounts of potassium and bicarbonate.

The preparation may also contain other naturally-occurring elements of the tear fluid, such as proteins, enzymes, lipids and metabolites as described in U.S. Patent No. 4,911,933. Typically, in an isotonic preparation, the potassium is present at a concentration of about 22.0 to 43.0 mM/l, the bicarbonate is present at a concentration of about 29.0 to 50.0 mM/l, the sodium is present at a concentration of about 130.0 to 140.0 mM/l, and the chloride is present at a concentration of about 118.0 to 136.5 mM/l, or the electrolyte components can be diluted to create hypotonic formulations where the ratios between the electrolyte concentrations remain unchanged..

The eye drop compositions can further optionally include calcium, magnesium and phosphate. In such embodiments, in isotonic preparations, the calcium is preferably present at a concentration of about 0.5 to 2.0 mM/l, the magnesium is preferably present at a concentration of about 0.3 to 1. 1 mM/l, and the phosphate is preferably present at a concentration of about 0.8 to 2.2 mM/l. For hypotonic formulations, the electrolyte components can be diluted to create hypotonic formulations where the ratios between the electrolyte concentrations remain unchanged.

Accordingly, in a particular embodiment, the invention provides an ophthalmic solution set forth in Table 2 of the Examples.

The pH of the ophthalmic preparation generally ranges from about 7.0 to 8. 0, as measured by, for example, a Fisher pH Accumet Model 600. However, this pH range need not be rigidly adhered to, and it may be desirable to alter pH outside of this range, for instance, to improve ophthalmic drug penetration through the ocular surface. In view of the teachings provided herein, those skilled in the art may employ other pH ranges.

The eye care compositions of the invention can be applied to the ocular surface by various methods known in the art. For example, the preparation can be applied topically to the ocular surface as eye drops or ointments. The preparation can also be applied using an eye cup so that the eye is bathed. The preparation can also be applied using a continuous or near continuous infusion device for ocular surface irrigation and/or wetting and/or drug delivery. The preparation can also be applied by release from a sustained-release carrier such as a sustained-release polymer, a liposome or a microcapsule. The preparation may also be applied by devices that spray solutions as required onto the surface of the eye.

The invention is further directed to methods of using the compositions described above to treat a subject, e.g., a subject having or at risk of having an infection, e.g., an infection of the eye or skin. The method comprises the step of applying the antimicrobial preparation described herein to the site of the infection, or site where an infection is likely to occur, or the site from which an infection might originate, for a time and under conditions effective for reducing the amount of bacteria present. In a specific embodiment, the time and conditions selected result in an at least about 1 log reduction in colony-forming units of the infecting bacteria after one minute of exposure to the antimicrobial preparation. In other embodiments, the application of the antimicrobial preparation for one minute results in an at least about 2, 3, 4 or 5 log reduction in colony-forming units.

The invention also provides methods of treating ocular disorders such as blepharitis, dry eye, eye inflammatory disorders, infectious conjunctivitis, and other ocular disorders that result from or are complicated by bacterial colonization or infection of the eye or surrounding tissue, by applying the preparations provided herein to the eye and/or surrounding tissue of a subject.

The invention also provides methods of treating infection of the ocular surface by applying the antimicrobial preparations provided herein to the eye of a subject. Exemplary infections that can be treated with the antimicrobial preparations provided herein include conjunctivitis, e.g., infectious conjunctivitis and corneal ulcers.

The invention also provides methods of preventing an eye infection in a subject having an eye surgery or procedure. These methods would comprise applying the antimicrobial preparation to the eye over a number of days preceding the surgery or procedure to reduce or eliminate the risk of developing an infection during the surgery or procedure. Exemplary procedures include cataract or LASIK surgery.

The invention also provides methods of maintaining low bacterial colony counts on punctal plugs that have been placed in patients for treatment. Exemplary punctal plugs include those manufactured by Odyssey Medical (Memphis, TN), and Eagle Vision (Memphis, TN).

Application of the antimicrobial preparations set forth herein can be by any one of a number of art recognized methods. For example, application can be by a applicator, such as a Qtip or pad, by drops from a dropper or bottle, or using a finger or fingers.

The antimicrobial preparations of the invention may be applied one or more times per day, and may be left in place as long as needed, depending on the intended indication. The number of days which a subject applies the antimicrobial preparation, and the duration of the application, will depend on the intent of treatment or on the location and severity infection, and efficacy of the preparations on a given infection. In certain embodiments, the antimicrobial preparation may be applied for a period of 30 seconds, 45 seconds, 1 minute, 2 minutes, 3 minutes, 4 minutes, 5 minutes, or longer. The antimicrobial preparation can be applied by release from a sustained release carrier such as a sustained-release polymer, a liposome or a microcapsule. The ordinary skilled physician would be able to effectively prescribe a treatment regimen that will be effective in treating or preventing an infection in an individual.

The methods described above may further include a rinsing step after a recommended period of exposure. This step preferably comprises a simple water rinse. The antimicrobial preparation may be rinsed from the area to which it was applied with ample water after application, e.g., with a hand, finger or any moist pad or cloth suitable for this purpose.

In further embodiments, the invention provides disinfecting a surface. Exemplary surfaces include those on medical instrument, in medical facilities, on medical devices, and those in a home, e.g., in a kitchen or bathroom.

### Commercial Applications

The methods and compositions of the invention find numerous commercial applications that could beneficially utilize compliance enhancing methods and compositions for antibacterial applications. Consequently, the invention includes a kit comprising the compositions of the invention, e.g., a kit for the treatment of an infection, e.g., an ocular infection, an ocular disorder, eyelid hygiene. The kits optionally include an applicator. The preparation can be in the form of drops, solution, paste, cream, foam, gel, or ointment, or the like, when included in the kits of the invention.

The kit may optionally be packaged with instructions for use. The kit may optionally contain a dispenser or applicator, e.g., a sponge, to apply the antimicrobial preparations of the invention to the infected area of a subject.

### EXAMPLES

A study was conducted to evaluate the stability of three different formulations of the eye care preparations comprising chlorine dioxide. The basic formulations were prepared with either 0.005% chlorine dioxide alone, or 0.005% chlorine dioxide and 2.5% dyphylline. The study incorporated an evaluation of each formulation with and without autoclave sterilization in the manufacturing procedure.

The analysis consisted of both USP Antimicrobial Effectiveness (preservative efficacy) testing and analytical testing over a three month time frame, at three storage conditions: 5°C, 25°C/60% relative humidity (RH), and 40°C/75% RH. Analytical testing for chlorine dioxide content, pH, and osmolality was performed every two weeks for three months. The Antimicrobial Effectiveness was evaluated on a monthly basis on formulations that met the acceptance criteria for a Category 1 article at the initial Antimicrobial Effectiveness testing.

The analytical testing of the dyphylline/chlorine dioxide samples showed a pH drift upward in both the autoclaved and non-autoclaved formulations.

The initial Antimicrobial Effectiveness testing indicated that both autoclaved and non-autoclaved dyphylline/chlorine dioxide samples met the USP criteria at initial formulation. Samples of the autoclaved dyphylline/chlorine dioxide formulation were then tested after four months (all conditions) and met USP criteria for Antimicrobial Effectiveness.

In the chlorine dioxide formulations without dyphylline, the chlorine dioxide concentration showed no clear trend. The chlorine dioxide only formulation samples did not meet the acceptance criteria for the Antimicrobial Effectiveness testing during the initial testing and this portion of the study was discontinued.

During the execution of the initial study, a confirmatory study was performed to confirm the passing Antimicrobial Effectiveness results for the autoclaved dyphylline/chlorine dioxide sample. In addition to this sample, an autoclaved chlorine dioxide formulation and a formula based on example 5, US Patent 6,024,954 (consistent with Refresh) were prepared. All three were tested using the USP Antimicrobial Effectiveness testing, with the dyphylline/chlorine dioxide and the formulation based on example 5, US Patent 6,024,954 (consistent with Refresh) passing through 28 days, and the chlorine dioxide alone sample again failing at day 7.

### INTRODUCTION

The following variations of the formulations were prepared for this stability evaluation:

**Table 1: Chlorine Dioxide Formulation**

| **Chemical** | **Percent** |
|---|---|
| CMC | 0.25% |
| Sodium Chloride | 0.22218% |
| Potassium Chloride | 0.07315% |
| Magnesium Chloride, Hexahydrate | 0.005% |
| Sodium Phosphate, Monobasic, Monohydrate | 0.0056% |
| Calcium Chloride, Dihydrate | 0.00644% |
| Boric Acid | 0.12555% |
| Sodium Borate, Decahydrate | 0.00008% |
| Sodium Bicarbonate | 0.109887% |
| Chlorine Dioxide | 0.005% |

**Table 2: Dyphylline/Chlorine Dioxide Formulation**

| **Chemical** | **Percent** |
|---|---|
| CMC | 0.25% |
| Sodium Chloride | 0.11542% |
| Potassium Chloride | 0.038% |
| Magnesium Chloride, Hexahydrate | 0.0026% |
| Sodium Phosphate, Monobasic, Monohydrate | 0.00291% |
| Calcium Chloride, Dehydrate | 0.0035% |
| Boric Acid | 0.06522% |
| Sodium Borate, Decahydrate | 0.00004% |
| Sodium Bicarbonate | 0.05708% |
| Chlorine Dioxide | 0.005% |
| Dyphylline | 2.5% |

Each formula was prepared in duplicate, with one formula prepared without autoclaving, and the other prepared with autoclaving.

When each formulation and initial analytical testing was completed, samples were submitted to microbiology for the initial Antimicrobial Effectiveness Testing. The challenge organisms were S. *aureus, P. aeruginosa, E. coli, C. albicans,* and *A. niger.*

The remainder of the bulk solutions was filled into low density polyethylene (LDPE) bottles and placed into the appropriate stability chambers. The autoclaved dyphylline/chlorine dioxide formulation had an additional test performed at approximately four months to correlate with the last Antimicrobial Effectiveness testing.

During the execution of the initial study, a confirmatory study was performed to confirm the passing Antimicrobial Effectiveness results for the autoclaved dyphylline/chlorine dioxide sample. In addition to this sample, an autoclaved chlorine dioxide formulation was prepared as well as a formula based on example 5, US Patent 6,024,954 (consistent with Refresh) were prepared (formulation in Table 3).

**Table 3: Formulation based on example 5, US Patent 6, 024,954 (consistent with Refresh)**

| **Chemical** | **Percent** |
|---|---|
| CMC | 0.50% |
| Sodium Chloride | 0.62% |
| Potassium Chloride | 0.14% |
| Magnesium Chloride; Hexahydrate | 0.006% |
| Calcium Chloride, Dihydrate | 0.02% |
| Boric Acid | 0.20% |
| Chlorine Dioxide | 0.005% |

Samples of each formula were submitted for USP Antimicrobial Effectiveness testing.

### EQUIPMENT

325=/-2.5°C Incubator, VWR S/N 1000590
22.5±/-2.5°C Incubator, Forma S/N 32889120
Spectrophotometer, Spectronic 2ODT, S/N
3DU9337006 Centrifuge, ID 34440967
pH Meter, ID 0157189
Osmometer, ID T09390

### MATERIALS

*1. Staphylococcus aurcus,* ATCC 6538
*2. Pseudomonas aeruginosa, ATCC* 9027
*3. Escherichia coil,* ATCC 8739
*4. Candida albicans, ATCC* 10231
*5. Aspergillus niger,* ATCC 16404
6. Soybean Casein Digest Agar, (SODA)
7. Sabouraud Dextrose Agar, (SDA)
8. D/E Neutralizing Broth, (DEB)
9. 0.1N Sodium Thiosulfate Volumetric Solution
10. Potassium Iodide
11. Starch Indicator Solution
12. 2.5N Hydrochloric Acid

### EXPERIMENTS

After compounding the six formulations, samples were submitted for the initial USP Antimicrobial Effectiveness testing per USP and SOP-00181. All test samples were challenged with approximately 1.0x10 to 1.0x10⁶ cfu/mT, of *S*. *aureus* ATCC 6538, *P. aeruginosa* ATCC 9027, *E*. *coli* ATCC 8739, *C*. *albicans, ATCC* 10231 and *A. niger, A TCC* 16404. The organisms were inoculated into a 50mL centrifuge tube containing 10mL of test sample at Time=O. One (1) mL was aliquoted from each centrifuge tube for the following 4 weeks. The log reduction was determined by the plate count method after 7 14, 21 and 28 days by diluting in DEB from 10⁻¹ to 10⁻⁴ for bacteria/yeast and 10⁻1 to 10⁻ for mold. The plates were then poured with the appropriate media and incubated. Bacterial plates were poured with SCDA and incubated at 32.5±2.5°C. Yeast/mold plates were poured with SDA and incubated at 22.5±2.5°C. The initial antimicrobial effectiveness data was reviewed before any further testing for antimicrobial effectiveness was conducted. Only formulas that had met the acceptance criteria at the initial testing were followed beyond the initial testing.

The acceptance criteria for USP Antimicrobial Effectiveness for a Category 1 item are:
Bacteria -
   7 day - Not less than 1.0 log reduction from initial count
   14 day - Not less than 3.0 log reduction from initial count
   21 day - No specification
   28 day - No increase from 14 days count at 28 days
Yeast/Molds -
   7 and 14 days - No increase from initial count
   21 days - No specification
   28 days - No increase from initial count

### (No increase is defined as NMT 0.5 log₁₀)

Samples of each formulation were pulled from all storage conditions every two weeks and analyzed for chlorine dioxide, pH, and osmolality.

### RESULTS

The analytical testing of the dyphylline/chlorine dioxide samples showed a pH drift upward in both the autoclaved and non-autoclaved formulations, with the higher temperatures demonstrating a more dramatic pH change (see Table 4). There were no apparent changes in osmolarity in all samples at all storage conditions (Table 5). The chlorine dioxide concentration in the dyphylline/chlorine dioxide samples showed no clear trend (Table 6).

For the antimicrobial effectiveness testing, the initial formulations (both autoclaved and non-autoclaved), met the acceptance criteria. For the last (four month) time point, samples of the autoclaved formulation at all storage conditions were tested for antimicrobial effectiveness. The samples of the autoclaved formulation stored at 5°C, 25°C%60%RH, and 40°C/75%RH all met the acceptance criteria for USP Antimicrobial Effectiveness after four months (see Tables 7-11).

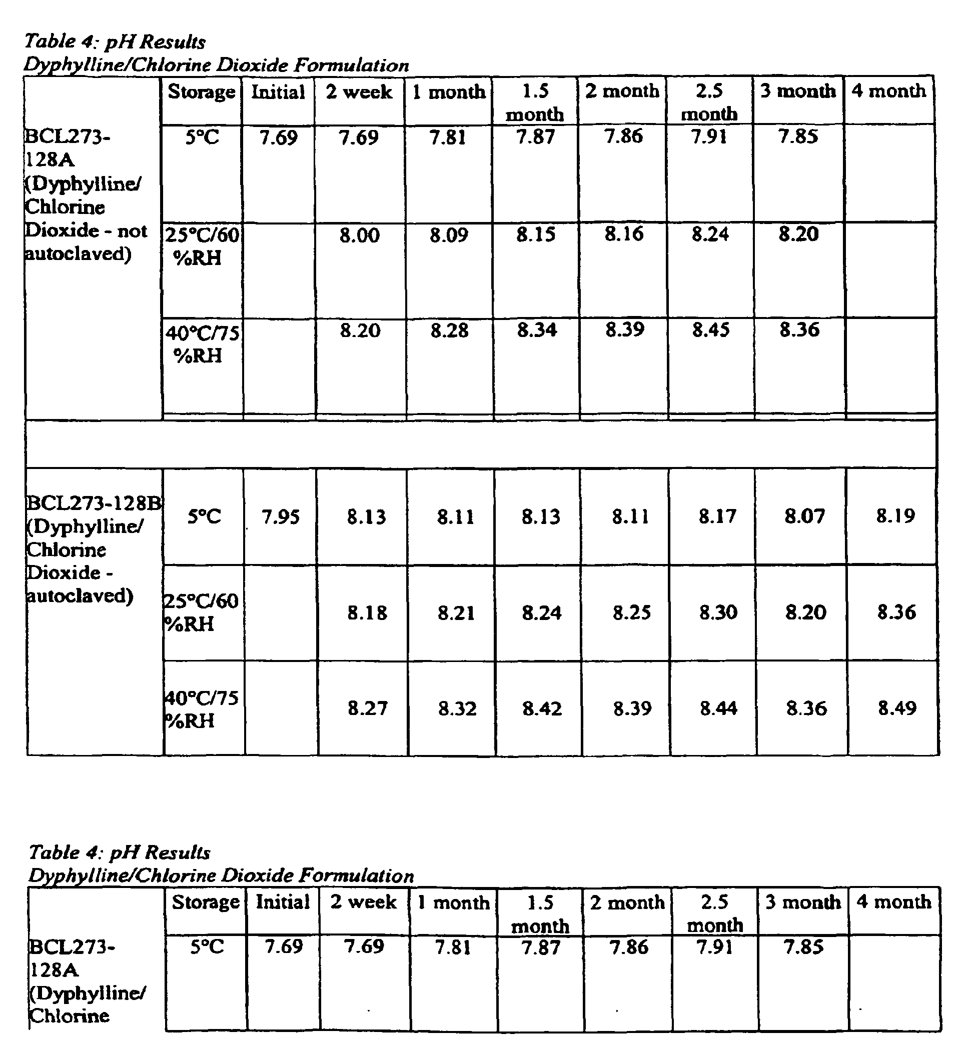

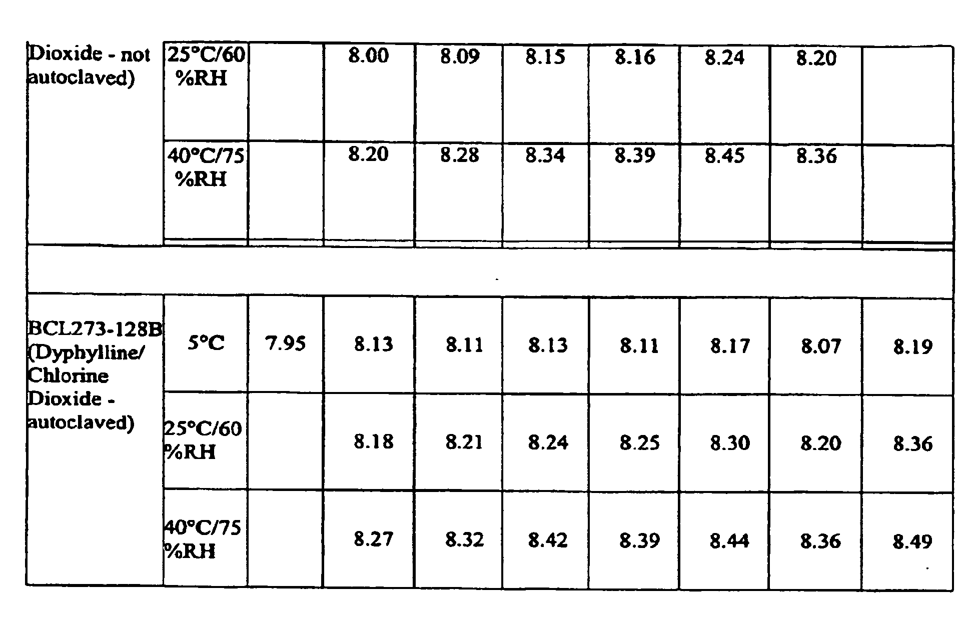

**Table 5: Osmolality Results (mOsm/1g)**

| *Dyphylline*/*Chlorine Dioxide Formulation* | | | | | | | | | |
|---|---|---|---|---|---|---|---|---|---|
| | Storage | Initial | 2 week | 1 month | 1.5 month | 2 month | 2.5 month | 3 month | 4 month |
| BCL273-128A (Dyphylline/ Chlorine Dioxide - not autoclaved) | 5°C | 156 | 157 | 157 | 158 | 156 | 153 | 154 | |
| | 25°C/60% RH | | 156 | 154 | 156 | 153 | 152 | 153 | |
| | 40°C/75% RH | | 154 | 155 | 154 | 151 | 151 | 151 | |
| | | | | | | | | | |
| BCL273-128B (Dyphylline/ Chlorine Dioxide-autoclaved) | 5°C | 157 | 159 | 160 | 161 | 157 | 157 | 159 | 159 |
| | 25°C/60% RH | | 157 | 158 | 159 | 156 | 156 | 159 | 158 |
| | 40°C/75% RH | | 158 | 157 | 158 | 155 | 154 | 155 | 156 |

**Table 6: Chlorine Dioxide Results (ppm) Dyphylline/Chlorine**

| *Dioxide Formulation* | | | | | | | | | |
|---|---|---|---|---|---|---|---|---|---|
| | Storage | Initial | 2 week | 1 month | 1.5 month | 2 month | 2.5 month | 3 month | 4 month |
| BCL273-128A (Dyphylline/ Chlorine Dioxide - not autoclaved) | 5°C | 59.14 | 62.52 | 62.52 | 59.14 | 60.83 | 59.14 | 59.14 | |
| | 25°C/60% RH | | 60.83 | 62.52 | 58.29 | 61.67 | 64.21 | 60.83 | |
| | 40°C/75% RH | | 61.67 | 63.36 | 59.14 | 60.83 | 57.45 | 59.98 | |
| | | | | | | | | | |
| BCL273-128B (Dyphylline/ Chlorine Dioxide -autoclaved) | 5°C | 59.98 | 62.52 | 59.14 | 60.83 | 62.52 | 62.52 | 67.58 | 65.05 |
| | 25°C/60% RH I | | 62.52 | 64.21 | 60.83 | 60.83 | 60.83 | 66.74 | 62.52 |
| | 40°C/75% RH | | 62.52 | 65.90 | 60.83 | 61.67 | 61.67 | 59.14 | 60.83 |

**Table 7: Antimicrobial Effectiveness**

| *Dyphylline*/*Chlorine Dioxide - not autoclaved, BCL273-128A, T= initial* | | | |
|---|---|---|---|
| Organism | Day | Log reduction | Pass/Fail |
| *S. aureus* | 7 | >4.94 | Pass |
| | 14 | >4.94 | Pass |
| | 21 | >4.94 | N/A |
| | 28 | >4.94 | Pass |
| *P. aeruginosa* | 7 | >5.07 | Pass |
| | 14 | >5.07 | Pass |
| | 21 | >5.07 | N/A |
| | 28 | >5.07 | Pass |
| *E. coli* | 7 | >4.96 | Pass |
| | 14 | >4.96 | Pass |
| | 21 | >4.96 | N/A |
| | 28 | >4.96 | Pass |
| *C. albicans* | 7 | 1.06 | Pass |
| | 14 | 1.04 | Pass |
| | 21 | 2.74 | N/A |
| | 28 | 3.58 | Pass |
| *A. niger* | 7 | 0.91 | Pass |
| | 14 | 1.00 | Pass |
| | 21 | 1.61 | N/A |
| | 28 | 1.05 | Pass |

**Table 8: Antimicrobial Effectiveness**

| *Dyphy*/*line*/*Chlo rine Dioxide - autoclaved. BCL273-128B, T= initial* | | | |
|---|---|---|---|
| Organism | Day | Log reduction | Pass/Fail |
| *S. aureus* | 7 | >4.94 | Pass |
| | 14 | >4.94 | Pass |
| | 21 | >4.94 | N/A |
| | 28 | >4.94 | Pass |
| *P. aeruginosa* | 7 | 4.47 | Pass |
| | 14 | >5.07 | Pass |
| | 21 | >5.07 | N/A |
| | 28 | >5.07 | Pass |
| *E. coli* | 7 | >4.96 | Pass |
| | 14 | >4.96 | Pass |
| | 21 | >4.96 | N/A |
| | 28 | >4.96 | Pass |
| *C. albicans* | 7 | 0.34 | Pass |
| | 14 | 1.04 | Pass |
| | 21 | 1.23 | N/A |
| | 28 | 1.97 | Pass |
| *A. niger* | 7 | 0.85 | Pass |
| | 14 | 1.05 | Pass |
| | 21 | 1.71 | N/A |
| | 28 | 1.64 | Pass |

**Table 9: Antimicrobial Effectiveness**

| *Dyphylline*/*Chlorine Dioxide autoclaved, BCL 273-128B. 40°C*/*75% RH Storage, T=4 month* | | | |
|---|---|---|---|
| Organism | Day | Log reduction | Pass/Fail |
| *S. aureus* | 7 | >5.07 | Pass |
| | 14 | >5.07 | Pass |
| | 21 | >5.07 | N/A |
| | 28 | >5.07 | Pass |
| *P. aeruginosa* | 7 | >5.06 | Pass |
| | 14 | >5.06 | Pass |
| | 21 | >5.06 | N/A |
| | 28 | >5.06 | Pass |
| *E. coli* | 7 | >5.09 | Pass |
| | 14 | >5.09 | Pass |
| | 21 | >5.09 | NIA |
| | 28 | >5.09 | Pass |
| *C. albicans* | 7 | 0.04 | Pass |
| | 14 | 1.80 | Pass |
| | 21 | 2.00 | N/A |
| | 28 | 2.29 | Pass |
| *A. niger* | 7 | 1.05 | Pass |
| | 14 | 1.08 | Pass |
| | 21 | 1.14 | N/A |
| | 28 | 1.35 | Pass |

**Table 10: Antimicrobial Effectiveness**

| *Dyphylline*/*Chlorine Dioxide -T= 4 month autoclaved. BCL 273-128B, 25°C*/*60% RH Storage,* | | | |
|---|---|---|---|
| Organism | Day | Log reduction | Pass/Fail |
| *S. aureus* | 7 | >5.07 | Pass |
| | 14 | >5.07 | Pass |
| | 21 | >5.07 | N/A |
| | 28 | >5.07 | Pass |
| *P. aeruginosa* | 7 | 4.46 | Pass |
| | 14 | >5.06 | Pass |
| | 21 | >5.06 | N/A |
| | 28 | >5.06 | Pass |
| *E. coli* | 7 | >5.09 | Pass |
| | 14 | >5.09 | Pass |
| | 21 | >5.09 | N/A |
| | 28 | >5.09 | Pass |
| *C. albicans* | 7 | 0.07 | Pass |
| | 14 | 0.98 | Pass |
| | 21 | 1.03 | N/A |
| | 28 | 1.37 | Pass |
| *A. niger* | 7 | 0.94 | Pass |
| | 14 | 0.94 | Pass |
| | 21 | 1.12 | N/A |
| | 28 | 1.20 | Pass |

**Table 11: Antimicrobial: Effectiveness**

| *Dyphylline*/*Chlorine Dioxide - autoclaved. BCL 173-118B, 5°C Storage, T= 4 month* | | | |
|---|---|---|---|
| Organism | Day | Log reduction | Pass/Fail |
| *S. aureus* | 7 | >5.07 | Pass |
| | 14 | >5.07 | Pass |
| | 21 | >5.07 | N/A |
| | 28 | >5.07 | N/A |
| *P. aeruginosa* | 7 | 3.81 | Pass |
| | 14 | >5.06 | Pass |
| | 21 | >5.06 | N/A |
| | 28 | >5.06 | Pass |
| *E. coli* | 7 | >5.09 | Pass |
| | 14 | >5.09 | Pass |
| | 21 | >5.09 | N/A |
| | 28 | >5.09 | Pass |
| *C. albicans* | 7 | 0.32 | Pass |
| | 14 | 0.94 | Pass |
| | 21 | 0.95 | N/A |
| | 28 | 1.24 | Pass |
| *A. niger* | 7 | 0.97 | Pass |
| | 14 | 1.05 | Pass |
| | 21 | 1.08 | N/A |
| | 28 | 1.35 | Pass |

### Chlorine Dioxide, autoclaved and non-autoclaved:

During the 3 months of testing, pH of both autoclaved and non-autoclaved increased in all conditions (see Table 12). There were no apparent changes in osmolality for all samples at all storage conditions (Table 13). The chlorine dioxide concentration showed no clear trend (see Table 14). These samples did not meet specification for USP Antimicrobial Effectiveness for the initial testing (Tables 15 and 16).

**Table 12: pH Results**

| *Chlorine Dioxide Formulation* | | | | | | | | |
|---|---|---|---|---|---|---|---|---|
| | Storage | Initial | 2 week | 1 month | 1.5 month | 2 month | 2.5 month | 3 month |
| BCL273-161C (Chlorine Dioxide - not autoclaved) | 5°C | 7.77 | 7.95 | 8.00 | 8.13 | 8.04 | 7.80 | 8.14 |
| | 25°C/60%RH | | 8.11 | 8.24 | 8.35 | 8.32 | 8.36 | 8.44 |
| | 40°C/75%RH | | 8.35 | 8.49 | 8.59 | 8.56 | 8.57 | 8.68 |
| | 55°C | | 8.55 | 8.68 | 8.78 | 8.73 | 8.69 | 8.84 |
| | | | | | | | | |
| BCL273-161D (Chlorine Dioxide - autoclaved) | 5°C | 8.54 | 8.51 | 8.49 | 8.54 | 8.43 | 8.37 | 8.49 |
| | 25°C/60%RH | | 8.53 | 8.54 | 8.58 | 8.50 | 8.45 | 8.50 |
| | 40°C/75%RH | | 8.58 | 8.64 | 8.70 | 8.64 | 8.60 | 8.73 |
| | 55°C | | 8.66 | 8.74. | 8.80 | 8.75 | 8.73 | 8.86 |

**Table 13: Osmolality, Results (mOsm/kg) Chlorine**

| *Dioxide Formulation* | | | | | | | | |
|---|---|---|---|---|---|---|---|---|
| | Storage | Initial | 2 week | 1 month | 1.5 month | 2 month | 2.5 month | 3 month |
| BCL273-161C (Chlorine Dioxide - not autoclaved) | 5°C | 141 | 142 | 140 | 138 | 141 | 138 | 136 |
| | 25°C/60%RH | | 141 | 136 | 136 | 138 | 136 | 134 |
| | 40°C/75%RH | | 139 | 135 | 134 | 134 | 135 | 134 |
| | 55°C | | 139 | 135 | 136 | 137 | 136 | 137 |
| | | | | | | | | |
| BCL273-161D (Chlorine Dioxide - autoclaved) | 5°C | 144 | 146 | 143 | 143 | 144 | 145 | 142 |
| | 25°C/60%RH | | 146 | 143 | 143 | 144 | 143 | 144 |
| | 40°C/75%RH | | 146 | 142 | 142 | 143 | 142 | 141 |
| | 55°C | | 145 | 143 | 141 | 145 | 150 | 148 |

**Table 14: Chlorine Dioxide Results (ppm)**

| *Chlorine Dioxide Formulation* | | | | | | | | |
|---|---|---|---|---|---|---|---|---|
| | Storage | Initial | 2 week | 1 month | 1-5 month | 2 month | 2.5 month | 3 month |
| CL273-1610 (Chlorine Dioxide) not autoclaved) | 5°C | 59.14 | 59.14 | 60.53 | 59.14 | 59.14 | 60.83 | 60.83 |
| | 25°C/60%RH | | 58.29 | 59.98 | 59.14 | 69.27 | 62.52 | 59.99 |
| | 40°C/75%RH | | 59.14 | 60.83 | 61.67 | 65.90 | 62.52 | 57.45 |
| | 55°C | 59.14 | | 60.83 | 64.21 | 65.90 | 6252 | 59.29 |
| | | | | | | | | |
| BCL273-161D (Chlorine Dioxide - autoclaved) | 5°C | 62.52 | 67.58 | 65.05 | 65.90 | 62.52 | 69.27 | 62.52 |
| | 25°C/60%RH | | 59.98 | 63.36 | 63.36 | 71.81 | 62.52 | 65.05 |
| | 40°C/75%RH | | 60.83 | 64.21 | 65.90 | 60.83 | 60.83 | 62.52 |
| | 55°C | | 60.83 | 62.52 | 67.58 | 64.21 | 62.52 | 64.21 |

**Table 15: Antimicrobial Effectiveness**

| *Chlorine Dioxide - not autoclaved, BCL273-161 C, T= initial* | | | |
|---|---|---|---|
| Organism | Day | Log reduction | Pass/Fail |
| *S. aureus* | 7 | >4.97 | Pass |
| | 14 | >4.97 | Pass |
| | 21 | Discontinued testing | |
| | 28 | | |
| *P. aeruginosa* | 7 | 0.78 | Fail |
| | 14 | 1.46 | N/A |
| | 21 | Discontinued testing | |
| | 28 | | |
| *E. coli* | 7 | 2.07 | Pass |
| | 14 | 3.16 | Pass |
| | 21 | Discontinued testing | |
| | 28 | | |
| *C. albicans* | 7 | 0.99 | Pass |
| | 14 | 1.94 | Pass |
| | 21 | Discontinued testing | |
| | 28 | | |
| *A. niger* | 7 | 1.81 | Pass |
| | 14 | 2.26 | Pass |
| | 21 | Discontinued testing | |
| | 28 | | |

**Table 16: Antimicrobial Effectiveness**

| *Chlorine Dioxide - autoclaved, BCL273-161D, T= initial* | | | |
|---|---|---|---|
| Organism | Day | Log reduction | Pass/Fail |
| *S. aureus* | 7 | >4.97 | Pass |
| | 14 | >4.97 | Pass |
| | 21 | Discontinued testing | |
| | 28 | | |
| *P. aeruginosa* | 7 | 0.88 | Fail |
| | 14 | 2.08 | N/A |
| | 21 | Discontinued testing | |
| | 28 | | |
| *E. coli* | 7 | 1.53 | Pass |
| | 14 | 2.76 | Fail |
| | 21 | Discontinued testing | |
| | 28 | | |
| *C. albicans* | 7 | 0.97 | Pass |
| | 14 | 2.09 | Pass |
| | 21 | Discontinued testing | |
| | 28 | | |
| *A. niger* | 7 | 1.85 | Pass |
| | 14 | 2.95 | Pass |
| | 21 | Discontinued testing | |
| | 28 | | |

During the execution of the initial study, a confirmatory study was performed to confirm the passing antimicrobial results for the autoclaved dyphylline/chlorine dioxide sample. In addition to this sample, an autoclaved chlorine dioxide formulation and a formulation based on example 5 of US Patent 6,024,954, consistent with the Refresh product marketed by Allergan were prepared for the confirmatory studies. All three were analyzed in the USP Antimicrobial Effectiveness test, and the earlier tests were confirmed with the dyphylline/chlorine dioxide and the formulation based on example 5, US Patent 6,024,954 (consistent with Refresh) passing through 28 days, and the chlorine dioxide alone sample failing at day 7 (see Tables 17-19).

**Table 17: Antimicrobial Effectiveness**

| *Repeated Dyphylline*/*Chlorine Dioxide - autoclaved, BCL285-040B. T= initial* | | | |
|---|---|---|---|
| Organism | Day | Log reduction | Pass/Fail |
| *S. aureus* | 7 | > 4.99 | Pass |
| | 14 | > 4.99 | Pass |
| | 21 | > 4.99 | N/A |
| | 28 | > 4.99 | Pass |
| *P. aeruginosa* | 7 | >5.14 | Pass |
| | 14 | >5.14 | Pass |
| | 21 | >5.14 | N/A |
| | 28 | >5.14 | Pass |
| *E. coli* | 7 | 4.77 | Pass |
| | 14 | >5.07 | Pass |
| | 21 | >5.07 | N/A |
| | 28 | 5.07 | Pass |
| *C. albicans* | 7 | 0.17 | Pass |
| | 14 | 0.76 | Pass |
| | 21 | 0.99 | N/A |
| | 28 | 1.87 | Pass |
| *A. niger* | 7 | 2.01 | Pass |
| | 14 | 1.35 | Pass |
| | 21 | 1.97 | N/A |
| | 28 | 2.28 | Pass |

**Table 18: Antimicrobial Effectiveness**

| *Repeated Chlorine Dioxide - autoclaved, BCL285-040A, T= initial* | | | |
|---|---|---|---|
| Organism | Day | Log reduction | Pass/Fail |
| *S. aureus* | 7 | > 4.99 | Pass |
| | 14 | > 4.99 | Pass |
| | 21 | > 4.99 | N/A |
| | 28 | > 4.99 | Pass |
| *P. aeruginosa* | 7 | 0.92 | Fail |
| | 14 | 2.20 | N/A |
| | 21 | 1.35 | N/A |
| | 28 | -0.44 | N/A |
| *E. coli* | 7 | 1.71 | Pass |
| | 14 | 1.96 | Fail |
| | 21 | 2.63 | N/A |
| | 28 | 2.51 | N/A |
| *C. albicans* | 7 | 0.97 | Pass |
| | 14 | 1.72 | Pass |
| | 21 | 4.13 | N/A |
| | 28 | >5.28 | Pass |
| *A. niger* | 7 | 2.18 | Pass |
| | 14 | 2.89 | Pass |
| | 21 | 3.18 | N/A |
| | 28 | 3.51 | Pass |

**Table 19: Antimicrobial Effectiveness**

| *Formula based on example 5.* US Patent 6,024,954 *(consistent with Refresh)* | | | |
|---|---|---|---|
| Organism | Day | Log reduction | Pass/Fail |
| *S. aureus* | 7 | > 4.99 | Pass |
| | 14 | 4.99 | Pass |
| | 21 | > 4.99 | N/A |
| | 28 | > 4.99 | Pass |
| *P. aeruginosa* | 7 | >5.14 | Pass |
| | 14 | >5.14 | Pass |
| | 21 | >5.14 | N/A |
| | 28 | >5.14 | Pass |
| *E. coli* | 7 | >5.07 | Pass |
| | 14 | >5.07 | Pass |
| | 21 | >5.07 | N/A |
| | 28 | >5.07 | Pass |
| *C. albicans* | 7 | >5.28 | Pass |
| | 14 | >5.28 | Pass |
| | 21 | >5.28 | N/A |
| | 28 | >5.28 | Pass |
| *A. niger* | 7 | 1.18 | Pass |
| | 14 | 1.10 | Pass |
| | 21 | 1.00 | N/A |
| | 28 | 1.31 | Pass |

### CONCLUSION

Samples of the dyphylline/chlorine dioxide formulation stored at 5°C, 25°C/60%RH and 40°C/75% RH met the criteria for USP Antimicrobial Effectiveness for a Category 1 item after four months of storage.

The chlorine dioxide only formulation samples did not meet the acceptance criteria for the USP Antimicrobial Effectiveness testing for a Category 1 item.

The confirmatory Antimicrobial Effectiveness testing did confirm the observed results of the initial Antimicrobial Effectiveness testing for the dyphylline/chlorine dioxide and chlorine dioxide formulations. The dyphylline/chlorine dioxide formulation again met the acceptance criteria for USP Antimicrobial Effectiveness for a Category 1 item. The chlorine dioxide formulation did not meet the acceptance criteria for USP Antimicrobial Effectiveness for a Category 1 item. During the same testing, the formulation based on example 5, US Patent 6,024,954 (consistent with Refresh) also met the acceptance criteria for USP Antimicrobial Effectiveness for a Category 1 item.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An ophthalmologic preparation comprising an oxidizing antibacterial agent and a heterocyclic compound that improves the antibacterial effect of the oxidizing agent, wherein said oxidizing antibacterial agent is chlorine dioxide, and wherein the heterocyclic compound is a xanthine.

2. The preparation of claim 1, wherein the heterocyclic compound is selected from the group consisting of caffeine, theophylline, dyphylline, theobromine, xanthine, xanthinol, methylxanthine, and aminophylline.

3. The preparation of claim 1 or 2, wherein the chlorine dioxide is present in a concentration of about 25-125 ppm; or wherein the chlorine dioxide is present in a concentration of about 50-75 ppm; or wherein the chlorine dioxide is present in a concentration of about 60 ppm.

4. The preparation of any preceding claim, wherein the preparation is in the form of a drop, solution, cream, paste, ointment, or gel; or wherein the preparation is incorporated into a sustained-release carrier, wherein, preferably, said sustained-release carrier is selected from the group consisting of a sustained-release polymer, a liposome and a microcapsule.

5. The preparation of any preceding claim, wherein the preparation is a solution, wherein, preferably, the preparation has an osmolality of 180 mOsm/Kg or less; or wherein, preferably, the preparation has an osmolality of 165 mOsm/Kg or less; or wherein the preparation has an osmolality of 165 mOsm/Kg or less and wherein the oxidizing antibacterial agent is chlorine dioxide which is present in a concentration of about 50-75 ppm.

6. The preparation of any preceding claim, wherein the preparation is for application to the eye, eye-lid, eye margin, punctal plug or a contact lens; or wherein the preparation is effective against at least one of the microbes selected from the group consisting of bacteria, fungi, and yeast; or wherein the preparation promotes an increase in conjunctival goblet- cell density in dry eye; or wherein the preparation promotes an increase in conjunctival goblet-cell density in eye surface inflammatory disorders.

7. The preparation of any preceding claim , further comprising a pharmaceutically acceptable carrier.

8. The preparation of claim 1, wherein the preparation is in the form of a solution comprising chlorine dioxide and dyphylline, wherein the chlorine dioxide is present at about 55-75 ppm and the antimicrobial preparation has a total osmolality of 165 mOsm/Kg or less; or wherein the preparation is a topical preparation comprising chlorine dioxide and dyphylline.

9. The preparation of claim 1, wherein the preparation is an eye care preparation comprising chlorine dioxide and dyphylline, wherein the chlorine dioxide is present at about 55-75 ppm, wherein, preferably, the eye care preparation is an eye drop in the form of a solution, or wherein, preferably, the eye care preparation in the form of a gel.

10. A preparation comprising a chlorite generating agent and dyphylline.

11. Use of the preparation of any preceding claim in the manufacture of a medicament for treating a microbial colonization or infection in a subject.

12. Use of the preparation of any one of claims 1-10 in the manufacture of a medicament for treating a colonization or infection of the ocular surface or eyelid in a subject.

13. The use of claim 12, wherein the infection is conjunctivitis; or wherein the infection is infectious conjunctivitis; or wherein the colonization or infection is of a punctal plug; or wherein the colonization or infection is in conjunction with dry eye; or wherein the colonization or infection is in conjunction with an eye surface inflammatory disorder; or wherein the colonization or infection is in conjunction with an eyelid disorder; or wherein the ocular surface is in contact with a contact lens.

14. A method of reducing the risk of contact lens colonization or infection by applying the preparation of any one of claims 1-10 to the surface of a contact lens prior to contact lens insertion in the eye.

15. Use of the preparation of any one of claims 1-10 in the manufacture of a medicament for any of the following:
a) reducing the risk of infection of the eye in an eye surgery patient prior to a surgical procedure;
b) treating colonization or an infection of the mucosal tissue or the epithelial tissue in a subject;
c) treating dry eye in a subject; or
d) increasing goblet cell density and mucosal epithelial membrane mucin expression in a subject.

## Patentansprüche

1. Ophthalmologische Zubereitung, die ein oxidierendes antibakterielles Mittel und eine heterocyclische Verbindung, die die antibakterielle Wirkung des oxidierenden Mittels verbessert, umfasst, worin genanntes oxidierendes antibakterielles Mittel Chlordioxid ist und worin die heterocyclische Verbindung ein Xanthin ist.

2. Zubereitung nach Anspruch 1, worin die heterocyclische Verbindung aus der Gruppe ausgewählt ist, die aus Folgenden besteht: Koffein, Theophyllin, Dyphyllin, Theobromin, Xanthin, Xanthinol, Methylxanthin und Aminophyllin.

3. Zubereitung nach Anspruch 1 oder 2, worin das Chlordioxid in einer Konzentration von etwa 25-125 ppm vorliegt; oder worin das Chlordioxid in einer Konzentration von etwa 50-75 ppm vorliegt; oder worin das Chlordioxid in einer Konzentration von etwa 60 ppm vorliegt.

4. Zubereitung nach einem vorstehenden Anspruch, worin die Zubereitung in Form von Tropfen, einer Lösung, Creme, Paste, Salbe oder eines Gels vorliegt; oder worin die Zubereitung in einem Träger mit verzögerter Freisetzung eingeschlossen ist, worin bevorzugt der genannte Träger mit verzögerter Freisetzung aus der Gruppe ausgewählt ist, die aus einem Polymer mit verzögerter Freisetzung, einem Liposom und einer Mikrokapsel besteht.

5. Zubereitung nach einem vorstehenden Anspruch, worin die Zubereitung eine Lösung ist, worin bevorzugt die Zubereitung eine Osmolalität von 180 mOsm/kg oder weniger hat; oder worin bevorzugt die Zubereitung eine Osmolalität von 165 mOsm/kg oder weniger hat; oder worin die Zubereitung eine Osmolalität von 165 mOsm/kg oder weniger hat und worin das oxidierende antibakterielle Mittel Chlordioxid ist, das in einer Konzentration von etwa 50-75 ppm vorliegt.

6. Zubereitung nach einem vorstehenden Anspruch, worin die Zubereitung zur Anwendung am Auge, Augenlid, Augenrand, Tränenwegsstöpsel oder an einer Kontaktlinse vorgesehen ist; oder worin die Zubereitung gegen mindestens eine der Mikroben, die aus der Gruppe ausgewählt ist, die aus Bakterien, Pilzen und Hefe besteht, wirksam ist; oder worin die Zubereitung eine Zunahme der Dichte an Becherzellen der Bindehaut bei einem trockenen Auge fördert; oder worin die Zubereitung eine Zunahme der Dichte an Becherzellen der Bindehaut bei entzündlichen Störungen an der Augenoberfläche fördert.

7. Zubereitung nach einem vorstehenden Anspruch, die weiter einen pharmazeutisch verträglichen Träger umfasst.

8. Zubereitung nach Anspruch 1, worin die Zubereitung in Form einer Lösung vorliegt, die Chlordioxid und Dyphyllin umfasst, worin das Chlordioxid zu etwa 55-75 ppm vorliegt und die antimikrobielle Zubereitung eine gesamte Osmolalität von 165 mOsm/kg oder weniger hat; oder worin die Zubereitung eine topische Zubereitung ist, die Chlordioxid und Dyphyllin umfasst.

9. Zubereitung nach Anspruch 1, worin die Zubereitung eine Zubereitung zur Augenpflege ist, die Chlordioxid und Dyphyllin umfasst, worin das Chlordioxid zu etwa 55-75 ppm vorliegt, worin bevorzugt die Zubereitung zur Augenpflege Augentropfen in Form einer Lösung sind oder worin bevorzugt die Zubereitung zur Augenpflege in Form eines Gels [vorliegt].

10. Zubereitung, die ein Chlorit-erzeugendes Mittel und Dyphyllin umfasst.

11. Verwendung der Zubereitung nach einem vorstehenden Anspruch bei der Herstellung eines Medikaments zur Behandlung einer mikrobiellen Kolonisierung oder Infektion bei einem Subjekt.

12. Verwendung der Zubereitung nach einem der Ansprüche 1-10 bei der Herstellung eines Medikaments zur Behandlung einer Kolonisierung oder Infektion der okularen Oberfläche oder des Augenlids bei einem Subjekt.

13. Verwendung nach Anspruch 12, worin die Infektion Bindehautentzündung ist; oder worin die Infektion infektiöse Bindehautentzündung ist; oder worin die Kolonisierung oder Infektion eines Tränenwegsstöpsels vorliegt; oder worin die Kolonisierung oder Infektion im Zusammenhang mit einem trockenen Auge steht; oder worin die Kolonisierung oder Infektion im Zusammenhang mit einer entzündlichen Störung an der Augenoberfläche steht; oder worin die Kolonisierung oder Infektion im Zusammenhang mit einer Störung am Augenlid steht; oder worin die okulare Oberfläche im Kontakt mit einer Kontaktlinse ist.

14. Verfahren zur Reduzierung des Risikos der Kolonisierung von Kontaktlinsen oder Infektion durch Auftragen der Zubereitung nach einem der Ansprüche 1-10 auf die Oberfläche einer Kontaktlinse vor dem Einsetzen der Kontaktlinse in das Auge.

15. Verwendung der Zubereitung nach einem der Ansprüche 1-10 bei der Herstellung eines Medikaments zu einem der folgenden Zwecke:
a) Reduzierung des Risikos einer Infektion des Auges bei einem Patienten für eine Augenoperation vor dem chirurgischen Eingriff;
b) Behandlung von Kolonisierung oder einer Infektion des Schleimhautgewebes oder des Epithelgewebes bei einem Subjekt;
c) Behandlung eines trockenen Auges bei einem Subjekt; oder
d) Zunahme der Dichte der Becherzellen und der Expression von Mucin der mukosalen epithelialen Membran bei einem Subjekt.

## Revendications

1. Préparation ophtalmologique comprenant un agent antibactérien oxydant et un composé hétérocyclique qui améliore l'effet antibactérien de l'agent oxydant, dans laquelle ledit agent antibactérien oxydant est le dioxyde de chlore, et dans laquelle le composé hétérocyclique est une xanthine.

2. Préparation selon la revendication 1, dans laquelle le composé hétérocyclique est choisi dans le groupe constitué par la caféine, la théophylline, la dyphylline, la théobromine, la xanthine, le xanthinol, la méthylxanthine et l'aminophylline.

3. Préparation selon la revendication 1 ou 2, dans laquelle le dioxyde de chlore est présent selon une concentration d'environ 25-125 ppm ; ou dans laquelle le dioxyde de chlore est présent selon une concentration d'environ 50-75 ppm ; ou dans laquelle le dioxyde de chlore est présent selon une concentration d'environ 60 ppm.

4. Préparation selon l'une quelconque des revendications précédentes, dans laquelle la préparation est sous la forme de gouttes, d'une solution, d'une crème, d'une pâte, d'un onguent ou d'un gel ; ou dans laquelle la préparation est incorporée dans un véhicule à libération prolongée, où, préférablement, ledit véhicule à libération prolongée est choisi dans le groupe constitué par un polymère à libération prolongée, un liposome et une microcapsule.

5. Préparation selon l'une quelconque des revendications précédentes, dans laquelle la préparation est une solution dans laquelle, préférablement, la préparation possède une osmolalité de 180 mOsm/kg ou moins ; ou dans laquelle, préférablement, la préparation possède une osmolalité de 165 mOsm/kg ou moins ; ou dans laquelle la préparation possède une osmolalité de 165 mOsm/kg ou moins et dans laquelle l'agent antibactérien oxydant est le dioxyde de chlore qui est présent selon une concentration d'environ 50-75 ppm.

6. Préparation selon l'une quelconque des revendications précédentes, dans laquelle la préparation est destinée à une application à l'oeil, la paupière, le bord de l'oeil, les bouchons lacrymaux ou une lentille de contact ; ou dans laquelle la préparation est efficace contre au moins un parmi les microbes choisis dans le groupe constitué par les bactéries, les champignons et les levures ; ou dans laquelle la préparation favorise une augmentation de la densité des cellules caliciformes conjonctivales dans l'oeil sec ; ou dans laquelle la préparation favorise une augmentation de la densité des cellules caliciformes conjonctivales dans les troubles inflammatoires de la surface de l'oeil.

7. Préparation selon l'une quelconque des revendications précédentes, comprenant en outre un véhicule pharmaceutiquement acceptable.

8. Préparation selon la revendication 1, dans laquelle la préparation est sous la forme d'une solution comprenant du dioxyde de chlore et de la dyphylline, dans laquelle le dioxyde de chlore est présent selon une concentration d'environ 55-75 ppm et la préparation antimicrobienne possède une osmolalité totale de 165 mOsm/kg ou moins ; ou dans laquelle la préparation est une préparation topique comprenant du dioxyde de chlore et de la dyphylline.

9. Préparation selon la revendication 1, dans laquelle la préparation est une préparation de soin des yeux comprenant du dioxyde de chlore et de la dyphylline, dans laquelle le dioxyde de chlore est présent selon une concentration d'environ 55-75 ppm, dans laquelle, préférablement, la préparation de soin des yeux est constituée de gouttes ophtalmiques sous forme sous la forme d'une solution, ou dans laquelle, préférablement, la préparation de soin des yeux [est] sous la forme d'un gel.

10. Préparation comprenant un agent générateur de chlorite et de la dyphylline.

11. Utilisation de la préparation selon l'une quelconque des revendications précédentes, dans l'élaboration d'un médicament destiné au traitement d'une infection ou d'une colonisation microbienne chez un sujet.

12. Utilisation de la préparation selon l'une quelconque des revendications 1-10, dans l'élaboration d'un médicament destiné au traitement d'une colonisation ou d'une infection de la surface oculaire ou de la paupière chez un sujet.

13. Utilisation selon la revendication 12, dans laquelle l'infection est la conjonctivite ; ou dans laquelle l'infection est la conjonctivite infectieuse ; ou dans laquelle la colonisation ou l'infection concerne un bouchon lacrymal ; ou dans laquelle la colonisation ou l'infection est en rapport avec un oeil sec ; ou dans laquelle la colonisation ou l'infection est en rapport avec un trouble inflammatoire de la surface de l'oeil ; ou dans laquelle la colonisation ou l'infection est en rapport avec un trouble de la paupière ; ou dans laquelle la surface oculaire est en contact avec une lentille de contact.

14. Méthode de réduction du risque d'infection ou de colonisation d'une lentille de contact, par l'application de la préparation selon l'une quelconque des revendications 1-10 à la surface de la lentille de contact préalablement à l'insertion de la lentille dans l'oeil.

15. Utilisation de la préparation selon l'une quelconque des revendications 1-10, dans l'élaboration d'un médicament destiné à un quelconque parmi ce qui suit :
a) la réduction du risque d'infection de l'oeil chez un patient subissant une chirurgie de l'oeil, préalablement à une opération chirurgicale ;
b) le traitement d'une colonisation ou d'une infection du tissu mucosal ou du tissu épithélial chez un sujet ;
c) le traitement de l'oeil sec chez un sujet ; ou
d) l'augmentation de la densité des cellules caliciformes et de l'expression de mucine des membranes épithéliales mucosales chez un sujet.
